(19) Europäisches Patentamt
European Patent Office
Office européen des brevets

(11) EP 3 687 966 B1

(12) # EUROPÄISCHE PATENTSCHRIFT

(45) Veröffentlichungstag und Bekanntmachung des Hinweises auf die Patenterteilung:
**03.01.2024 Patentblatt 2024/01**

(21) Anmeldenummer: **18773187.2**

(22) Anmeldetag: **24.09.2018**

(51) Internationale Patentklassifikation (IPC):
***C07C 41/03*** (2006.01) ***C07C 43/13*** (2006.01)
***C07D 303/24*** (2006.01) ***C09D 5/00*** (2006.01)
***C09D 7/40*** (2018.01) ***C09D 7/61*** (2018.01)

(52) Gemeinsame Patentklassifikation (CPC):
(C-Sets verfügbar)
**C07D 303/24; C07C 41/03; C07C 43/137; C09D 5/006; C09D 7/40; C09D 7/61** (Forts.)

(86) Internationale Anmeldenummer:
**PCT/EP2018/075725**

(87) Internationale Veröffentlichungsnummer:
**WO 2019/063452 (04.04.2019 Gazette 2019/14)**

(54) **VERFAHREN ZUR HERSTELLUNG VON FLUORIERTEN VERBINDUNGEN**

METHOD FOR PRODUCING FLUORINATED COMPOUNDS

PROCÉDÉ DE PRÉPARATION DE COMPOSÉS FLUORÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.09.2017 EP 17193044**

(43) Veröffentlichungstag der Anmeldung:
**05.08.2020 Patentblatt 2020/32**

(73) Patentinhaber: **Merck Patent GmbH**
**64293 Darmstadt (DE)**

(72) Erfinder:
- **FRIEDRICH, Reiner**
**64342 Seeheim-Jugenheim (DE)**
- **KOCH, Fabian**
**64319 Pfungstadt (DE)**

(74) Vertreter: **Merck Patent Association**
**Merck Patent GmbH**
**64271 Darmstadt (DE)**

(56) Entgegenhaltungen:
**WO-A1-2015/124290 WO-A1-2016/096129**

- **D. SOLOV'EV, L. KOLOMENSKAYA, A. RODIN: "Fluorine-containing 2,3-epoxypropyl ethers. Synthesis and spectral characteristics", THE JOURNAL OF GENERAL CHEMISTRY OF THE USSR (ENGL. TRANSL.), Bd. 61, 1991, Seiten 611-615, XP009509481, ISSN: 0022-1279**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C07C 41/03, C07C 43/137**

**Beschreibung**

Gegenstand der vorliegenden Erfindung ist ein Verfahren zur Herstellung von fluorierten Verbindungen, neue Verbindungen mit fluorierten Endgruppen, deren Verwendung und Mittel enthaltend neue Verbindungen mit fluorierten Endgruppen.

Fluorverbindungen stellen einen wichtigen Bestandteil in industriellen Prozesschemikalien dar. Fluorhaltige Verbindungen sind in verschiedensten Anwendungen einsetzbar und tragen z.B. zur verbesserten Benetzung von Oberflächen bei. So werden sie z.B. als Grenzflächenvermittler bzw. Emulgator oder Viskositätsminderer in Farben, Lacken oder Klebstoffen verwendet oder in schmutzabweisenden Beschichtungen, z. B. in der Textilindustrie.

Klassische Fluorverbindungen sind aus langkettigen, perfluorierten Alkylketten (C6-C8) aufgebaut und gelten potentiell als bioakkumulativ und toxisch. Aufgrund ihrer Persistenz und Toxizität sind diese Materialien problematisch für Anwender und Umwelt.

Kürzerkettige Fluorbausteine sind von ihren ökotoxikologischen Profilen günstiger, zeigen jedoch in ihren Anwendungsbereichen oft schlechtere Eigenschaften.

Auch verzweigte Fluortenside sind seit langem bekannt. Aufgrund der bisherigen Syntheseverfahren verfügen die Fluorketten bei verzweigten Fluorverbindungen immer über die gleiche Kettenlänge. Es besteht daher Bedarf an neuen fluorhaltigen Verbindungen und an Verfahren zur Herstellung von Verbindungen, die unterschiedliche fluorhaltige Gruppen enthalten.

Solov'ev et al., The Journal of General Chemistry of the USSR 1991, 61, 611-615, WO 2016/069129 A1 und WO 2015/124290 A1 beschreiben polyfluorierte niedermolekulare Verbindungen, offenbaren jedoch keine Verbindungen oder Verfahren wie in der vorliegenden Anmeldung beschrieben oder beansprucht.

Gegenstand der vorliegenden Erfindung ist ein Verfahren nach Anspruch 1 zur Herstellung fluorierter Verbindungen, fluorierte Verbindungen der Formeln (I') und (VI‘) nach Anspruch 7 bzw. 12, sowie deren Verwendungen und Mittel nach Anspruch 11, 15 und 16.

Durch die vorliegende Erfindung ist es nun möglich nicht nur verzweigte fluorierte Verbindungen mit gleichen fluorierten Endgruppen herzustellen sondern auch verzweigte Fluorverbindungen und fluorhaltige, funktionalisierbare Verbindungen mit asymmetrischem Aufbau.

Mit dem erfindungsgemäßen Verfahren nach Anspruch 1 können ausgehend von bekannten, kommerziell erhältlichen Verbindungen verzweigte, fluorhaltige Verbindungen der Formel (I) hergestellt werden, die unterschiedliche fluorhaltigen Gruppen, z. B. fluorhaltige Alkylketten mit unterschiedlichen Kettenlängen, enthalten. Das neue Verfahren eignet sich auch zur Herstellung von "gemischtverzweigten" Verbindungen, d.h. Verbindungen, die sowohl fluorhaltige als auch fluorfreie Gruppen enthalten. Ebenso kann das Verfahren auch zur Herstellung von verzweigten Verbindungen mit gleichen fluorhaltigen Gruppen herangezogen werden.

Die verzweigten Alkohole der Formel (I) können dann zu den entsprechenden fluorierten Tensiden, z. B anionischen bzw. nicht-ionischen Tensiden, oder fluorierten Acrylaten oder Silanen umgesetzt werden.

Die Herstellung von fluorierten Materialien mit unterschiedlich langen Ketten hat mehrere Vorteile. So lassen sich die Eigenschaften bezüglich CMC und Oberflächenspannungsreduktion in einem weiteren Umfeld manipulieren als es bei gleichlangen Ketten möglich ist.

In lösemittelhaltigen Lacksystemen wird oft die Kombination von fluorierten und nicht-fluorierten Kohlenwasserstofftensiden empfohlen. Durch die Kombination von fluorierten und nicht-fluorierten Ketten in einem Molekül können somit Synergien erwartet werden, die bei Verwendung reiner Fluortenside so nicht möglich sind.

Ein erster Gegenstand der vorliegenden Erfindung ist ein Verfahren nach Anspruch 1 zur Herstellung fluorierter Verbindungen der Formel (I)

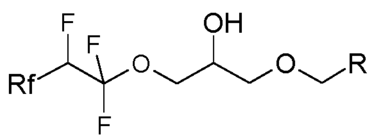

(I)

wobei

Rf eine fluorierte, lineare oder verzweigte, ggf. weitere Heteroatome enthaltende, Alkylgruppe ist,
R eine lineare oder verzweigte, ggf. Heteroatome enthaltende, Alkyl- oder Siloxangruppe oder eine Gruppe Rf‘ ist, und
Rf‘ eine fluorierte, lineare oder verzweigte, ggf. weitere Heteroatome enthaltende Alkylgruppe ist.

Nach dem Verfahren wird in einem ersten Schritt eine Verbindung der Formel (II), in der Rf eine fluorierte, lineare oder verzweigte, ggf. weitere Heteroatome enthaltende, Alkylgruppe ist, hergestellt

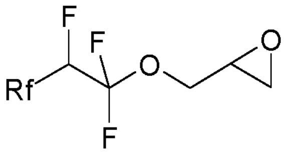

(II)

und diese dann in an sich bekannter Weise zu Verbindungen der Formel (I) umgesetzt.

Bevorzugt umfasst das erfindungsgemäße Verfahren die folgenden Schritte, wobei Rf, R und Rf‘ die für die Formeln (I) und (II) genannten Bedeutungen haben:

a) Umsetzen von Verbindungen der Formel (III) mit Glycidol (IV) zu Verbindungen der Formel (II)

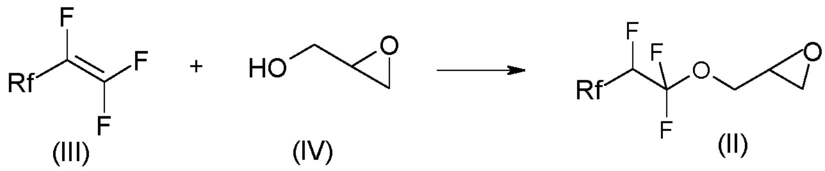

(III) (IV) (II)

und

b) Umsetzen von Verbindungen der Formel (II) mit Alkoholen der Formel (V) zu Verbindungen der Formel (I)

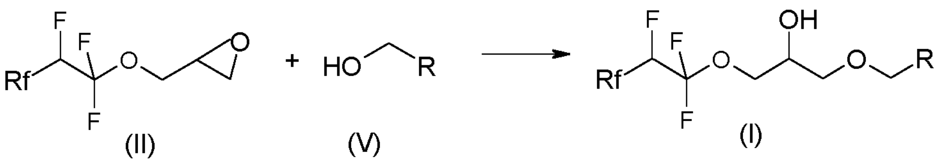

(II) (V) (I)

Bevorzugt ist Rf eine Gruppe der Formel

$$CF_3\text{-}(CF_2)_a\text{-}O_b\text{-}(CF_2)_c\text{-}O_d\text{-},$$

wobei

a = 0, 1, 2 oder 3,
b = 0 oder 1,
c = 0, 1, 2 oder 3 und
d = 0 oder 1 ist.

Bevorzugt ist Rf‘ eine Gruppe der Formel

$$CF_3\text{-}(CF_2)_a\text{-}O_b\text{-}(CF_2)_c\text{-}O_d\text{-}(CR^1R^2)_e\text{-}(CR^3R^4)_f\text{-}O_g\text{-},$$

wobei

$R^1$, $R^2$, $R^3$ und $R^4$ unabhängig voneinander Wasserstoff oder eine Alkylgruppe sind,
a = 0, 1, 2 oder 3,
b = 0 oder 1,
c = 0, 1, 2 oder 3,
d = 0 oder 1,
e = 0, 1, 2, 3 oder 4,
f = 0, 1, 2, 3 oder 4 und
g = 0 oder 1 ist.

Die Gruppen Rf und/oder Rf‘ können bevorzugt eine fluorierte C1-C6-Alkylgruppe sein, besonders bevorzugt eine perfluorierte C1-C4-Alkylgruppe, insbesondere eine perfluorierte C3-Alkylgruppe.

Bevorzugt sind die Gruppen Rf und Rf' nicht gleich.

In einer Variante des Verfahrens können die Gruppen Rf und Rf' aber auch gleich sein.

Bei der Verwendung von siloxanhaltigen Alkoholen bietet sich besonders die Verwendung des 1-Propanol, 3-[1,3,3,3 tetramethyl-1-[(trimethylsilyl)oxy]-1-disiloxanyl]- (Va) an, da diese Verbindung kommerziell erhältlich ist und in zahlreichen Tensidanwendungen eingesetzt wird.

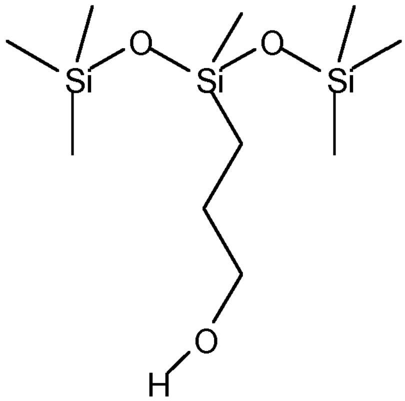

Va

Bevorzugt werden die erfindungsgemäßen Verfahrensschritte a) und b) wie folgt durchgeführt:
Die Umsetzung zu Formel (II) erfolgt dabei vorzugsweise in einem inerten Lösungsmittel (bevorzugt Ether z.B. Dioxan oder THF), einer Base (insbesondere Carbonate oder Hydroxyde der Alkali- oder Erdalkalimetalle, bevorzugt $K_2CO_3$ oder NaOH), dem entsprechenden Fluorolefin und dem Glycidol.

Das Glycidol wird dabei bevorzugt in einem stöchiometrischen bzw. leicht unterstöchiometrischen (1:1-1:0,85) Verhältnis zum Fluorolefin eingesetzt. Letzteres erleichtert die Aufarbeitung und verbessert die Ausbeuten. Das Reaktionsgemisch wird in einem Druckreaktor, insbesondere bei 80 - 140°C, bevorzugt 110 °C, bis zum Abflachen des Druckabfalls (in der Regel 12- 24h) zur Reaktion gebracht. Die Aufarbeitung erfolgt durch wässriges Ausschütteln und Trocknen der organischen Phase. Die Ausbeuten liegen zwischen 60-95% bezogen auf das Glycidol. Die teilfluorierten Epoxide lassen sich unter reduziertem Druck destillativ gut aufreinigen.

Zur weiteren Umsetzung werden Epoxide und die entsprechenden Alkohole mit einem Katalysatorsystem zur Reaktion gebracht. Dabei verwendet man bevorzugt eine Mischung aus $NaBH_4$, NaI und $I_2$ wie unter US 5608116 beschrieben.

Alternativ dazu kann die Reaktion mit elementarem Na oder Bortrifluoriddiethylether durchgeführt werden. Dazu wird der Alkohol vorgelegt, das Reagenz unter inerten Bedingungen und Kühlung zugesetzt und das Epoxid langsam zu dosiert. Nach beendeter Zugabe wird der Ansatz langsam auf 80°C erwärmt und mehrere Stunden intensiv verrührt. Als Alkohol zur additiven Ringöffnung eignen sich hier neben fluorierten Alkoholen mit C1-C6 auch rein aliphatische Alkohole oder Etheralkohole mit einer Gesamtkettenlänge von C1-C20, bevorzugt C6-C14. Möglich sind aber auch anderen H acide Verbindungen wie z.B. Siloxane oder Thiole.

Gegenstand der vorliegenden Erfindung sind auch Verbindungen der Formel (I') gemäß Anspruch 7,

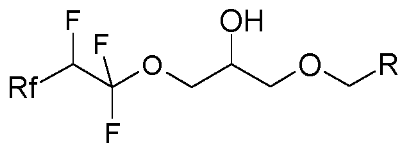

(I')

wobei

Rf eine fluorierte, lineare oder verzweigte, ggf. weitere Heteroatome enthaltende, Alkylgruppe ist,
R eine lineare oder verzweigte, ggf. Heteroatome enthaltende, Alkyl- oder Siloxangruppe oder eine Gruppe Rf' ist,
Rf' eine fluorierte, lineare oder verzweigte, ggf. weitere Heteroatome enthaltende Alkylgruppe ist,
und, wenn R eine Gruppe Rf' ist, Rf und Rf' verschieden sind.

Bevorzugt sind Verbindungen der Formel (I'), in denen Rf eine Gruppe der Formel $CF_3-(CF_2)_{0-3}-$ oder $CF_3-(CF_2)_{0-3}-O-$ ist.

Besonders bevorzugt ist Rf eine Gruppe der Formel $CF_3-(CF_2)_a-O_b-(CF_2)_c-O_d-$ mit a = 1 oder 2, b = 1, c = 0 und d = 0.

Bevorzugt kann die Gruppe R eine C1-C20 Alkylgruppe, insbesondere eine C6-C14 Alkylgruppe, oder eine Siloxangruppe sein.

Rf' ist bevorzugt eine Gruppe der Formel

$$CF_3-(CF_2)_a-O_b-(CF_2)_c-O_d-(CR^1R^2)_e-(CR^3R^4)_f-O_g-$$

wobei

$R^1$, $R^2$, $R^3$ und $R^4$ unabhängig voneinander Wasserstoff oder eine Alkylgruppe sind,
a = 0, 1, 2 oder 3,
b = 0 oder 1, insbesondere 1
c = 0, 1, 2 oder 3, insbesondere 0
d = 0 oder 1, insbesondere 0
e = 0, 1, 2, 3 oder 4, insbesondere 0 und 1
f = 0, 1, 2, 3 oder 4 insbesondere 0und
g = 0 oder 1 insbesondere 0 ist.

Besonders bevorzugt sind Verbindungen der Formel (I'), in denen Rf und Rf' die bevorzugten Bedeutungen haben.

Des Weiteren bevorzugt ist R = $CH_2-CH_2-Si\ (CH_3)-[O-Si(CH_3)_3]_2$.

Ein weiterer Gegenstand der Erfindung ist die Verwendung von Verbindungen der Formel (I) zur Herstellung von Verbindungen der Formel (VI):

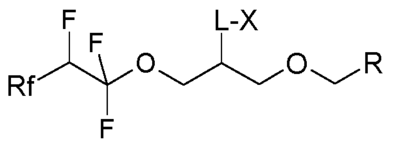

(VI)

wobei

Rf eine fluorierte, lineare oder verzweigte, ggf. weitere Heteroatome enthaltende, Alkylgruppe ist,
R eine lineare oder verzweigte, ggf. Heteroatome enthaltende, Alkyl- oder Siloxangruppe oder eine Gruppe Rf' ist,
Rf' eine fluorierte, lineare oder verzweigte, ggf. weitere Heteroatome enthaltende Alkylgruppe ist,
L eine Einfachbindung oder eine bivalente organische Gruppe ist und X eine kationische, nichtionische, amphotere oder anionische Gruppe ist.

Bevorzugt haben die Gruppen Rf, R, und Rf' die für die Formel (I) genannten Bedeutungen, insbesondere die bevorzugten Bedeutungen.

Die Einführung der hydrophilen, anionischen, kationischen, reaktiven oder polymerisierbaren Endgruppe ist dem Fachmann nach bekannten Methoden möglich.

Als Beispiel für nicht ionischen Tenside wäre hier Polyethylenglykol oder Polypropylenglykol bzw. deren Copolymere zu nennen, die sich aus den entsprechenden monomeren Epoxiden herstellen lassen. Vorteilhaft bei diesen Verbindungen ist, dass sich über die Kettenlänge der sog. HLB-Wert der Verbindung einstellen lässt.

Durch Umsetzung des Alkohols mit Glycidyltrimethylammoniumchlorid erhält man kationische Tenside.

Eine einfache Methode zur Herstellung von anionischen Tensiden ist die Herstellung sogenannter Phosphorsäureester. Dabei wird der fluorierte Alkohol mit $P_2O_5$ bzw. $POCl_3$ umgesetzt und mit einer Base neutralisiert. Eine weitere Möglichkeit zur Herstellung von anionischen Tensiden ist die Umsetzung des Alkohols mit $SO_3$ bzw. konzentrierte Schwefelsäure zu sogenannten Sulfaten.

Als polymerisierbare Gruppen sind besonders die (Meth)acrylate zu nennen, die aus den entsprechenden Säurechloriden bzw. Anhydriden gewonnen werden können.

Gegenstand der vorliegenden Erfindung sind auch Verbindungen der Formel (VI') gemäß Anspruch 12:

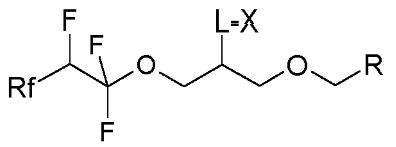

(VI')

wobei

Rf eine fluorierte, lineare oder verzweigte, ggf. weitere Heteroatome enthaltende, Alkylgruppe ist,
R eine C1-C20 Alkylgruppe ist,
L eine Einfachbindung oder eine bivalente organische Gruppe ist und
X eine kationische, nichtionische, amphotere oder anionische Gruppe ist.

Bevorzugt enthalten die Verbindungen der Formel (VI') eine Gruppe Rf mit der Formel $CF_3-(CF_2)_{0-3}-$ oder $CF_3-(CF_2)_{0-3}-O-$.

Bevorzugt kann die Gruppe R eine C6-C14 Alkylgruppe sein.

Besonders bevorzugt sind Verbindungen der Formel (VI'), in denen Rf die bevorzugten Bedeutungen hat.

Eine bevorzugte anionische Gruppe X kann ausgewählt sein aus $-COO^-$, $-SO_3^-$, $-OSO_3^-$, $-PO_3^{2-}$, $-OPO_3^{2-}$, $-(OCH_2CH_2)_s-O-(CH_2)_t-COO^-$, $-(OCH_2CH_2)_s-O-(CH_2)_t$ $-SO_3^-$, $-(OCH_2CH_2)_s-O-(CH_2)_t-OSO_3^-$, $-(OCH_2CH_2)_s-O-(CH_2)_t-PO_3^{2-}$, $-(OCH_2CH_2)_s-O-(CH_2)_t-OPO_3^{2-}$wobei s für eine ganze Zahl aus dem Bereich von 1 bis 1000 und t für eine ganze Zahl ausgewählt aus 1, 2, 3 oder 4 steht.

Zu den bevorzugten anionischen Gruppen gehören dabei insbesondere $-COO^-$, $-SO_3^-$, $-OSO_3^-$, $-PO_3^{2-}$, $-OPO_3^{2-}$, die Teilformel A, sowie $-(OCH_2CH_2)_s-O-(CH_2)_t-COO^-$, $-(OCH_2CH_2)_s-O-(CH_2)_t-SO_3^-$und $-(OCH_2CH_2)_s-O-(CH_2)_t-OSO_3^-$, wobei jede einzelne dieser Gruppen für sich genommen bevorzugt sein kann. X kann auch für die entsprechenden Säuren stehen.

Zu den ganz besonders bevorzugten anionischen Gruppen gehören dabei $-SO_3^-$, $-OSO_3^-$, $-COO^-$, $-PO_3^{2-}$, oder $OPO_3^{2-}$. Insbesondere eine Sulfonatgruppe $-SO_3^-$ ist bevorzugt.

Bevorzugtes Gegenion für anionische Gruppen X ist ein einwertiges Kation, insbesondere $H^+$, ein Alkalimetall-Kation oder $NR_4^+$, wobei R = H oder C1 - C6-Alkyl ist und alle R gleich oder verschieden sein können. Insbesondere bevorzugt sind $H^+$, $Na^+$, $K^+$, $Li^+$ und $NH_4^+$, besonders bevorzugt $Na^+$.

Eine bevorzugte kationische Gruppe X kann ausgewählt sein aus $-NR^1R^2R^{3+}$ $Z^-$, $-PR^1R^2R^{3+}$ $Z^-$,

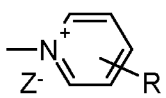
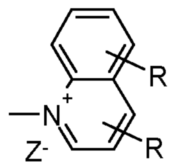
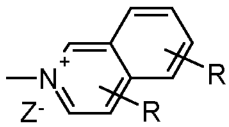

wobei R steht für H oder $C_{1-4}$-Alkyl in beliebiger Position,
Z- steht für $Cl^-$, Br-, $I^-$, $CH_3SO_3^-$, $CF_3SO_3^-$, $CH_3PhSO_3^-$, $PhSO_3^-$
$R^1$, $R^2$ und $R^3$ jeweils unabhängig voneinander stehen für H, $C_{1-30}$-Alkyl, Ar oder $-CH_2Ar$ und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können.

Zu den bevorzugten kationischen Gruppen gehören dabei insbesondere $-NR^1R^2R^{3+}$ $Z^-$ und

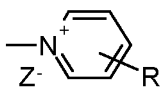

wobei jede einzelne dieser Gruppen für sich genommen bevorzugt sein kann.

Eine bevorzugte nicht-ionische Gruppe X kann ausgewählt sein aus:

lineares oder verzweigtes Alkyl, wobei ein oder mehrere nicht benachbarte C-Atome durch O, S, und/oder N ersetzt sind, -OH, -SH, $-O-(Glycosid)_{o'}$, $-S-(Glycosid)_{o'}$, $-OCH_2-CHOH-CH_2-OH$, $-O$ $CH_2Ar(-NCO)_{p'}$, $-OAr(-NCO)_{p'}$, Aminoxid,

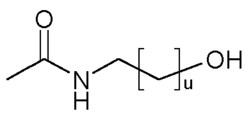 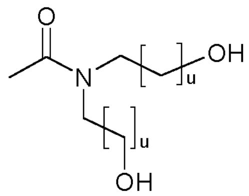 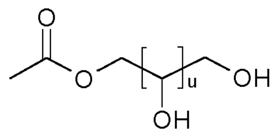

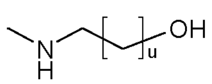 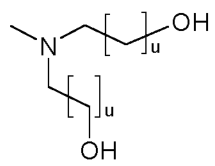 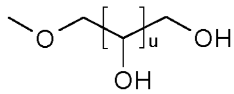

u steht für eine ganze Zahl aus dem Bereich von 1 bis 6, bevorzugt 1 bis 4
o' steht für eine ganze Zahl aus dem Bereich von 1 bis 10,
p' steht für 1 oder 2,
Ar steht für einen unsubstituierten, ein- oder mehrfach substituierten aromatischen Ring oder kondensierte Ringsysteme mit 6 bis 18 C-Atomen,
worin auch ein oder zwei CH-Gruppen durch C=O ersetzt sein können und, Glycosid steht für ein verethertes Kohlenhydrat, vorzugsweise für ein mono- di-, tri- oder oligo-Glucosid.

Zu den bevorzugten nicht-ionischen Gruppen X gehören dabei insbesondere lineares oder verzweigtes Alkyl, wobei ein oder mehrere nicht benachbarte C-Atome durch O, S und/oder N ersetzt sind, -OH und -O-(Glycosid)$_{o'}$.

Wenn X = Alkyl, wobei ein oder mehrere nicht benachbarte C-Atome durch O, S, und/oder N ersetzt sind, dann ist es bevorzugt gleich $R^4$-(B-A)$_{m''}$- mit $R^4$ = H oder C1-4-Alkyl, insbesondere H oder $CH_3$, A = lineares oder verzweigtes Alkylen, bevorzugt mit 1 bis 10 Kohlenstoffatomen, insbesondere mit 1 bis 4 Kohlenstoffatomen, B = O oder S, bevorzugt O, und m" = eine ganze Zahl bevorzugt aus dem Bereich von 1 bis 100, besonders bevorzugt 1 bis 30.

Insbesondere bevorzugt als nicht-ionische Gruppe X ist die Gruppe $R^4$-(O-$CH_2CHR^5$)$_{m''}$- mit m" = eine ganze Zahl aus dem Bereich von 1 bis 100, bevorzugt 1 bis 30, insbesondere auch 1-25, und $R^4$ und $R^5$ = H oder C1-4-Alkyl, insbesondere H oder $CH_3$. Insbesondere bevorzugt ist $R^4$-(B-A)$_{m''}$- eine Polyethylen- oder Polypropylenglykoleinheit.

Insbesondere bevorzugt als nicht-ionische Gruppe X ist die Gruppe -CH(OH)-$CH_2$-NH-Sach mit Sach = verschiedene Zucker und die Gruppe -Y-($CH_2$-$CH_2$-O)$_v$-$R^4$ mit Y = S, O oder NH, bevorzugt O, $R^4$ = H oder Alkyl, bevorzugt H oder $CH_3$, und v = 1-100, bevorzugt 1-30, insbesondere auch 1-25.

Eine bevorzugte amphotere Gruppe X kann ausgewählt sein aus den funktionellen Gruppen der Acetyldiamine, der N-Alkylaminosäuren, der N-Alkylaminosulfonsäuren, der Betaine, der Sulfobetaine, bzw. entsprechender Derivate, insbesondere ausgewählt aus, wobei M steht für H oder ein Alkalimetall-Ion, vorzugsweise $Li^+$, $Na^+$ oder $K^+$:

| |
|---|
| 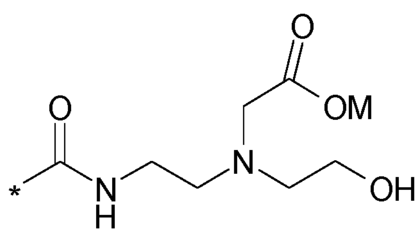 |
| 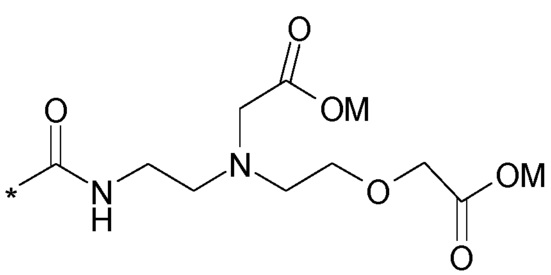 |

(fortgesetzt)

| |
|---|
| 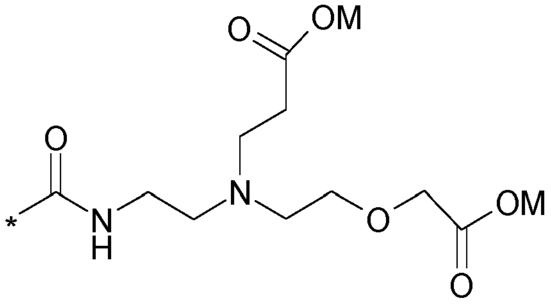 |
| $-NH-CH_2-COOM$; $-NH-CH_2-CH_2-COOM$ |
| 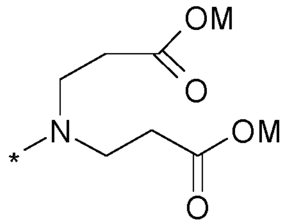 |
| $-[(C(=O)-NH-(CH_2)_{(1-8)}]_{(0\ oder\ 1)}-N^+R^1R^2-CH_2-COO^-$, wobei $R^1$ und $R^2$ jeweils unabhängig voneinander stehen für einen C1-8-Alkylrest, vorzugsweise Methyl oder Ethyl |
| $-C(=O)-NH-(CH_2)_{1-3}-N^+R^1R^2-CH_2-CH(OH)-CH_2-(O)(_{0\ oder\ 1)}-(S\ oder\ P)O_3^-$, wobei $R^1$ und $R^2$ jeweils unabhängig voneinander stehen für einen C1-8-Alkylrest, vorzugsweise Methyl oder Ethyl |

Besonders bevorzugte erfindungsgemäße Verbindungen sind solche, die als hydrophile Gruppe X eine der bevorzugten anionische Gruppen, der bevorzugten nicht-ionischen Gruppen oder der bevorzugten zwitterionischen Gruppen enthalten.

Insbesondere bevorzugt sind Verbindungen, die die Gruppen $-SO_3^-$, $-OSO_3^-$, $-COO^-$, $-PO_3^{2-}$ oder $OPO_3^{2-}$, Polyethylen- oder Polypropylenglykole, $-CH(OH)-CH_2-NH-Sach$, $-Y-(CH_2-CH_2-O)_v-R^4$, Betaine, oder Sulfobetaine enthalten. Bevorzugte Gegenionen sind hierbei $H^+$, $Na^+$, $K^+$ und $NH_4^+$, insbesondere $Na^+$. Insbesondere bevorzugt sind: $-SO_3^-$, $-COO^-$, Polyethylen- oder Polypropylenglykole, Sulfobetaine, die Gruppe $-CH(OH)-CH_2-NH-Sach$ und die Gruppe $-Y-(CH_2-CH_2-O)_v-R^4$ Hierbei ist Sach = verschiedene Zucker und Y = S, O oder NH, bevorzugt O, $R^4$ = H oder Alkyl, bevorzugt H oder $CH_3$, und v = 1-100, bevorzugt 1-30, insbesondere auch 1-25. Besonders vorteilhaft können auch Verbindungen mit X = $-SO_3^-$ sein.

Vorteile der erfindungsgemäßen Verbindungen der Formel (VI‘) können insbesondere sein:

- eine Oberflächenaktivität, die der konventioneller Kohlenwasserstoff-Tenside hinsichtlich Effizienz und/oder Effektivität gleich oder überlegen ist,
- biologische und/oder abiotische Abbaubarkeit der Substanzen ohne Bildung persistenter perfluorierter Abbauprodukte wie PFOA (Perfluoroctansäure) oder PFOS (Perfluoroctansulfonat),
- nach einfachen Verfahren herstellbar,
- schwache Schaumwirkung und/oder geringe Schaumstabilisierung,
- gute Verarbeitbarkeit in Formulierungen und/oder
- Lagerstabilität.

Bevorzugt können die erfindungsgemäßen Verbindungen eine besondere Oberflächenaktivität aufweisen. Die erfindungsgemäßen Verbindungen der Formel (VI‘), insbesondere die bevorzugten Verbindungen, können außerdem verbesserte Umwelteigenschaften aufweisen, da sie weder chemisch noch biologisch zu langkettigen PFCAs oder PFASs abbauen. Bevorzugt können die erfindungsgemäßen Verbindungen durch entsprechende Umwelteinflüsse vollständig in mineralisierbare/regenerierbare Verbindungen überführt werden.

Die Verwendung von Verbindungen der Formel (VI‘) und Mittel enthaltend Verbindungen der Formel (VI‘) sind ebenfalls Gegenstand der Erfindung.

Die Verbindungen der Formel (VI‘) können bevorzugt als oberflächenaktive Mittel verwendet werden, bevorzugt als Tensid, Hydrophobiermittel, Grenzflächenvermittler, Viskositätsminderer, Schaumstabilisator oder Emulgator. Ein

weiterer Gegenstand der vorliegenden Erfindung ist somit die Verwendung der erfindungsgemäßen Verbindungen und den vorstehend beschriebenen bevorzugten Ausführungsformen als oberflächenaktive Mittel, beispielsweise zur Verbesserung des Verlaufsverhaltens und des Benetzungsvermögens von Coatingformulierungen, insbesondere der genannten besonders bevorzugten Verbindungen.

Neben den Verbindungen der Formel (VI') können die erfindungsgemäßen Mischungen auch Lösemittel, Additive, Hilfs- und Füllstoffe sowie nicht fluorierte Tenside enthalten. Beispielhaft seien genannt Silikonpartikel, Weichmacher und oberflächenmodifizierte Pigmente.

Bevorzugte Einsatzgebiete sind beispielsweise die Verwendung der erfindungsgemäßen Fluortenside der Formel (VI') als Additive in Zubereitungen zur Oberflächenbeschichtung, wie Farben, Lacken, Schutzanstrichen, Spezialcoatings in elektronischen oder Halbleiter-Anwendungen (z.B. Photolacken, Top Antireflective Coatings, Bottom Antireflective Coatings) oder in optischen Anwendungen (z.B. photographischen Beschichtungen, Beschichtungen optischer Elemente), in Agrochemikalien, in Poliermitteln und Wachsen, z.B. für Mobiliar, Fußböden und Automobile, insbesondere in Bodenpolituren, in Feuerlöschmitteln, Schmierstoffen, in photolithographischen Verfahren, insbesondere in Immersionsphotolithographie-Verfahren, z.B. in Entwicklerlösungen, Spüllösungen, Immersionsölen und/oder in den Photoresists selbst, vor allem zur Herstellung von gedruckten Schaltungen oder in Additivzubereitungen zur Additivierung entsprechender Zubereitungen. Darüber hinaus eignen sich die erfindungsgemäß als Tensid verwendbaren Verbindungen für Wasch- und Reinigungsanwendungen, sowie für eine Verwendung als Additive/Tenside in kosmetischen Produkten wie z. B. Haar- und Körperpflegeprodukten (z.B. Shampoos, Haarspülungen und Haarkonditionierern), Schaumbädern, Cremes oder Lotionen mit einer oder mehreren der folgenden Funktionen: Emulgatoren, Netzmittel, Schaummittel, Gleitmittel, Antistatikum, Erhöher der Resistenz gegen Hautfette.

Dabei werden die erfindungsgemäßen Fluortenside für die Anwendung üblicherweise in entsprechend ausgelegten Zubereitungen eingebracht. Übliche Einsatzkonzentrationen sind 0.01 - 1.0 Gew.-% der erfindungsgemäßen Tenside bezogen auf die Gesamtzubereitung.

Entsprechende Mittel, enthaltend die erfindungsgemäßen Fluortensiden, sind ebenfalls Gegenstand der vorliegenden Erfindung. Bevorzugt enthalten solche Mittel einen für den jeweiligen Verwendungszweck geeigneten Träger, sowie gegebenenfalls weitere Aktivstoffe und/oder gegebenenfalls Hilfsstoffe. Bevorzugte Mittel sind Farb- und Lackzubereitungen, Feuerlöschmittel, Schmierstoffe, Wasch- und Reinigungsmittel und Enteiser oder Entwicklerlösungen, Spüllösungen, Immersionsöle und Photoresists für photolithograqphische Verfahren, insbesondere für Immersionsphotolithographie-Verfahren und insbesondere zur Herstellung gedruckter Schaltungen, Agrochemikalien, Bodenpolituren, kosmetische Produkte, kosmetische Produkte oder Hydrophobiermittel zur Textilausrüstung oder Glasbehandlung. Bei bevorzugten Mitteln handelt es sich dabei um Farb- und Lackzubereitungen und Druckfarben.

Außerdem sind auch wasserbasierte Lackformulierungen, die die erfindungsgemäßen Fluortenside allein oder im Gemische mit Additiven enthalten, Gegenstand der vorliegenden Erfindung. Bevorzugt werden Lackformulierungen auf Basis der folgenden synthetischen Filmbildner verwendet: Polykondensationsharze wie Alkydharze, gesättigt/ungesättigte Polyester, Polyamide/imide, Silikonharze; Phenolharze; Harnstoffharze und Melaminharze, Polyadditionsharze wie Polyurethane und Epoxidharze, Polymerisationsharze wie Polyolefine, Polyvinylverbindungen und Polyacrylate.

Außerdem sind die erfindungsgemäßen Fluortenside auch zum Einsatz in Lacken auf Basis von Naturstoffen und modifizierten Naturstoffen geeignet. Bevorzugt sind Lacke auf Basis von Ölen, Polysacchariden wie Stärke und Cellulose als auch auf Basis von Naturharzen wie cyclischen Oligoterpenen, Polyterpenen und/oder Schellack.

Die erfindungsgemäßen Fluortenside können sowohl in physikalisch härtenden (Thermoplaste) als auch in vernetzenden (Elastomere und Duromere) wässrigen Lacksystemen verwendet werden. Bevorzugt verbessern die erfindungsgemäßen Fluortenside die Verlaufs- und Benetzungseigenschaften der Lacksysteme.

Alle hier genannten Verwendungen erfindungsgemäß einzusetzender Fluortenside, insbesondere der bevorzugten Verbindungen, sind Gegenstand der vorliegenden Erfindung. Die jeweilige Anwendung von Fluortenside zu den genannten Zwecken ist dem Fachmann bekannt, so dass der Einsatz der erfindungsgemäß einzusetzenden Fluortenside keine Probleme bereitet.

Die folgenden Beispiele erläutern die vorliegende Erfindung näher, ohne den Schutzbereich zu beschränken.

## Beispiele

## Abkürzungen

PPVE Perfluorpropylvinylether
MTBE tert-Butylmethylether
PPOL-1 2,2,3-Trifluoro-3-heptafluoropropyloxy-propan-1-ol

**Bestimmung der statischen Oberflächenspannung**

Es werden die statischen Oberflächenspannungen γ von wässrigen Tensidlösungen mit verschiedenen Konzentrationen c (Gramm pro Liter) bestimmt.

> Gerät: Tensiometer der Firma Dataphysics (Modell DCAT 11)
> Temperatur der Messlösungen: 20°±0,2°C
> Eingesetzte Messmethode: Messung der Oberflächenspannung mit der Wilhelmy Plattenmethode nach DIN EN 14370.
> Platte: Platin, Länge= 19,9mm

Bei der Plattenmethode wird die Oberflächen- bzw. Grenzflächenspannung der Tensidlösung aus der auf die benetzte Länge einer Platte wirkenden F F Kraft nach folgender Formel berechnet: $\gamma = \frac{F}{L \cdot \cos\theta} = \frac{F}{L}$

> γ= Grenz- oder Oberflächenspannung; F= auf die Waage wirkende Kraft;
> L= benetzte Länge (19,9 mm); θ= Kontaktwinkel)Die Platte besteht aus angerautem Platin und wird also optimal benetzt, so dass der Kontaktwinkel θ nahe bei 0° liegt. Der Term cos θ erreicht daher annähernd den Wert 1, so dass nur noch die gemessene Kraft und die Länge der Platte berücksichtigt werden müssen.

<u>Beispiel 1:</u>

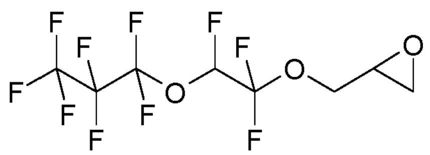

21.78 g Glycidol, 93,86 g PPVE, 12,19 g Kaliumcarbonat; 130 ml Dioxan Die Edukte werden in einem 300 mL-Druckreaktor zusammengegeben und für 24h bei 110C gerührt. Zu Beginn der Reaktion stellt sich ein Druck von 4,5bar ein, dieser fällt über Nacht auf 0,5bar ab. Das Reaktionsgemisch wird mit Wasser und MTBE versetzt und die Phasen getrennt. Die wässrige Phase wird mit 2x30mL MTBE extrahiert und die vereinigte organische Phase mit 40 mL Wasser und 40 mL gesättigter NaCl-Lösung gewaschen. Das Extrakt wird über Natriumsulfat getrocknet und das Lösemittel abdestilliert. Auswaage: 90,87 g Das Produkt wurde im Vakuum destilliert.

| $T_{Bad}$ °C | $T_{Kopf}$ °C | p mbar |
|---|---|---|
| 50,3 | 24,1 | 0,80 |

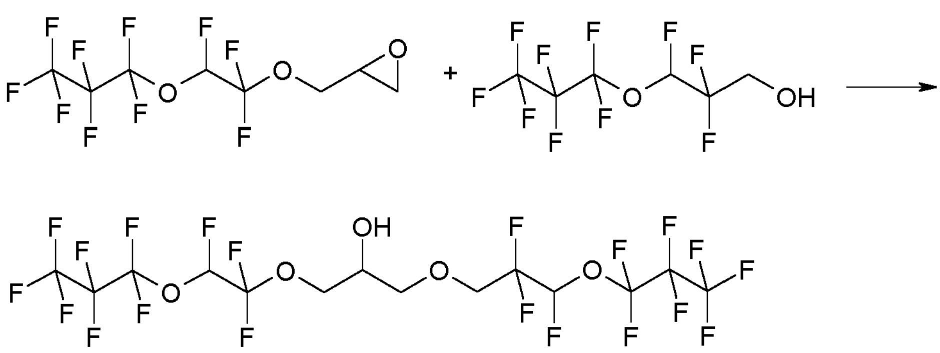

3,40 g Fluorepoxid aus Beispiel 1, 8,94 g Fluoralkohol (PPOL-1) und 0,36 g Natrium (elementar)

In einem Rundkolben wird PPOL-1 vorgelegt. Unter Kühlung wird elementares Natrium zugegeben. Zum vollständigen Lösen des Natriums wird das Reaktionsgemisch leicht erhitzt. Anschließend wird das Reaktionsgemisch wieder eingekühlt und das Epoxid zugegeben. Nach vollständiger Zugabe wird das Reaktionsgemisch für 20h bei 80 °C gerührt. Das Reaktionsgemisch wird mit MTBE und Wasser versetzt und die Phasen getrennt. Die wässrige Phase wurde mit 2x30mL MTBE extrahiert. Die vereinigte organische Phase wird mit jeweils 40 mL Wasser und 40 mL gesättigter NaCl-Lösung gewaschen. Das Extrakt wird über Natriumsulfat getrocknet und das Lösemittel abdestilliert. Auswaage: 15,04 g Das Produkt wird im Vakuum destilliert.

| p mbar | $T_{Bad}$ °C | $T_{Kopf}$ °C |
|---|---|---|
| 0,80 | 105,4 | 78,3 |

Auswaage: 4,7g

**Beispiel 2:**

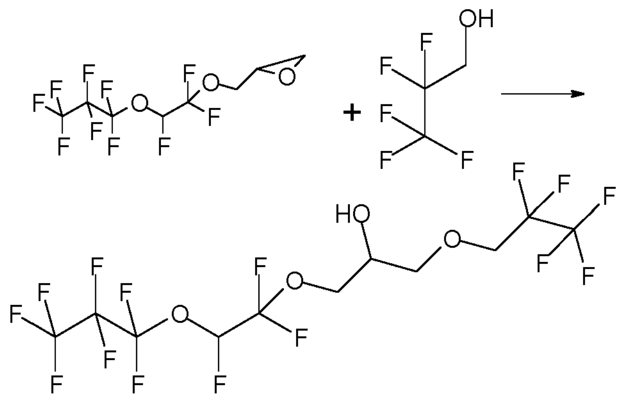

Die Edukte werden in einem 50mL-Druckreaktor zusammengegeben und langsam auf 130°C erhitzt. Nach 20h wird die Reaktion abgebrochen und das Reaktionsgemisch mit Wasser und MTBE versetzt. Die Phasen werden getrennt und die wässrige Phase wird mit 2x25mL MTBE extrahiert. Anschließend wird die vereinigte organische Phase mit jeweils 30mL Wasser und 30mL gesättigter NaCl-Lösung gewaschen. Das Extrakt wird über Natriumsulfat getrocknet und das Lösemittel abdestilliert. Das Reaktionsgemisch wird destillativ aufgereinigt. Auswaage: 6,55g
GC MS zeigt 82,77% Produkt und 6,81% Isomer.

**Beispiel 3:**

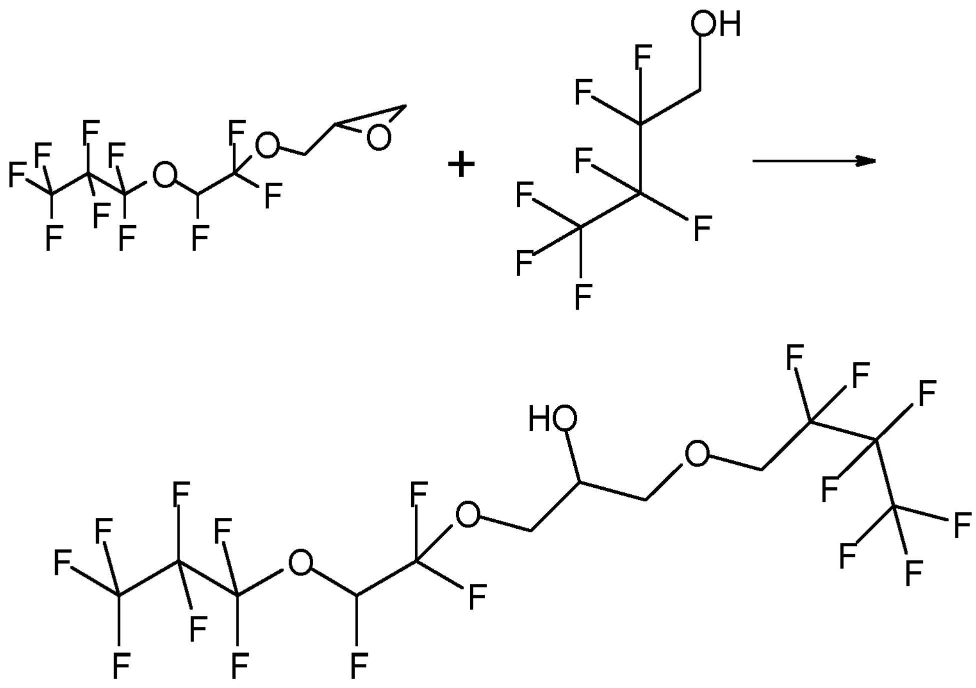

Die Edukte werden in einem 50mL-Druckreaktor zusammengegeben und langsam auf 130°C erhitzt. Nach 20h wird die Reaktion abgebrochen und das Reaktionsgemisch mit Wasser und MTBE versetzt. Die Phasen werden getrennt und die wässrige Phase wird mit 2x25mL MTBE extrahiert. Anschließend wird die vereinigte organische Phase mit jeweils 30mL Wasser und 30mL gesättigter NaCl-Lösung gewaschen. Das Extrakt wird über Natriumsulfat getrocknet und das Lösemittel abdestilliert.
GC-MS zeigt: 87,82% Produkt.

**Beispiel 4:**

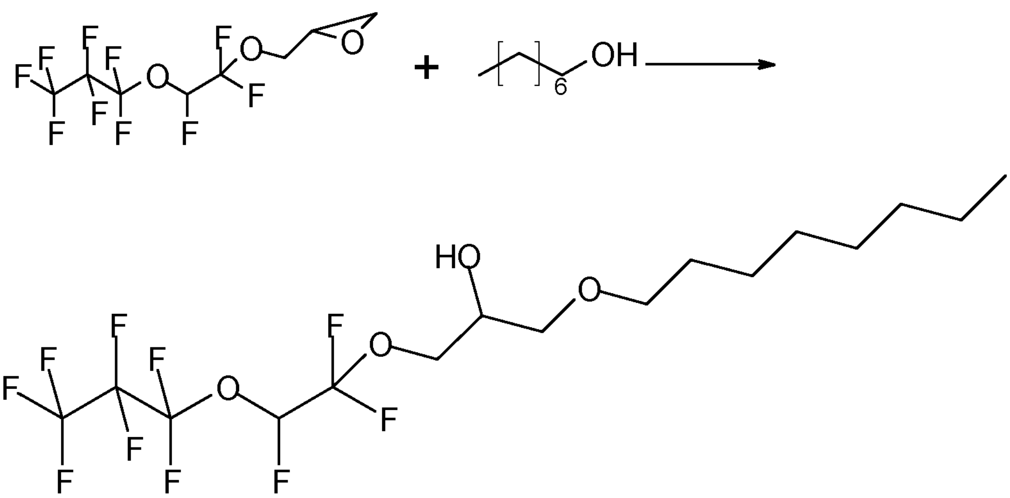

Die Edukte werden in einem 50mL-Druckreaktor zusammengegeben und langsam auf 130°C erhitzt. Nach 20h wird die Reaktion abgebrochen und das Reaktionsgemisch mit Wasser und MTBE versetzt. Die organische Phase zeigt eine Orangefärbung. Die Phasen werden getrennt und die wässrige Phase wird mit 2x25mL MTBE extrahiert. Anschließend wird die vereinigte organische Phase mit jeweils 30mL Wasser und 30mL gesättigter NaCl-Lösung gewaschen. Das Extrakt wird über Natriumsulfat getrocknet und das Lösemittel abdestilliert. GC-MS mittels PCI weist einen Massepeak von 488 auf. Dieser Peak entspricht etwa 50% der Peakflächen. Dies entspricht dem Produkt plus angelagertes Ammonium.

**Patentansprüche**

1. Verfahren zur Herstellung von Verbindungen der Formel (I)

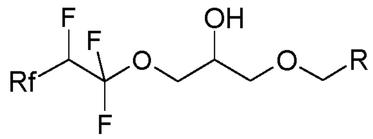

(I)

wobei zuerst Verbindungen der Formel (II)

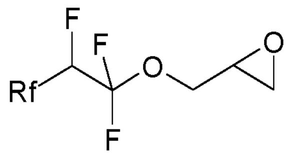

(II)

hergestellt werden und diese dann in an sich bekannter Weise zu Verbindungen der Formel (I) umgesetzt werden, wobei
Rf eine fluorierte, lineare oder verzweigte, ggf. weitere Heteroatome enthaltende, Alkylgruppe ist,
R eine lineare oder verzweigte, ggf. Heteroatome enthaltende, Alkyl- oder Siloxangruppe oder eine Gruppe Rf‘ ist, und
Rf‘ eine fluorierte, lineare oder verzweigte, ggf. weitere Heteroatome enthaltende Alkylgruppe ist.

2. Verfahren gemäß Anspruch 1 umfassend die Schritte:

a) Umsetzen von Verbindungen der Formel (III) mit Glycidol (IV) zu Verbindungen der Formel (II)

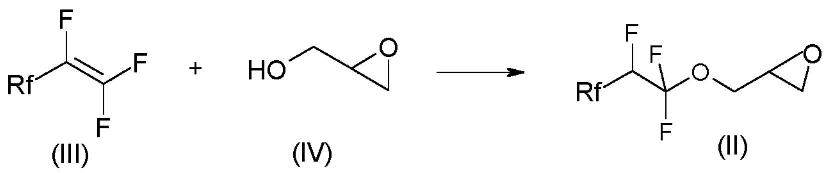

(III) (IV) (II)

und
b) Umsetzen von Verbindungen der Formel (II) mit Alkoholen der Formel (V) zu Verbindungen der Formel (I)

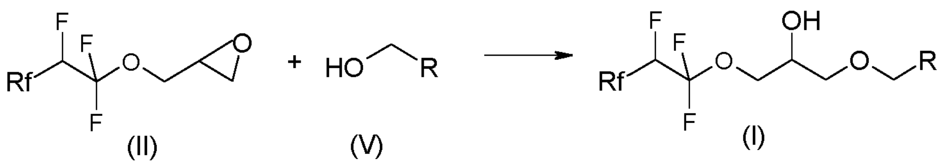

(II) (V) (I)

wobei

Rf eine fluorierte, lineare oder verzweigte, ggf. weitere Heteroatome enthaltende, Alkylgruppe ist,
R eine lineare oder verzweigte, ggf. Heteroatome enthaltende, Alkyl- oder Siloxangruppe oder eine Gruppe Rf‘ ist, und
Rf‘ eine fluorierte, lineare oder verzweigte, ggf. weitere Heteroatome enthaltende Alkylgruppe ist.

3. Verfahren gemäß einem oder mehreren der Ansprüche 1 bis 2, **dadurch gekennzeichnet, dass** Rf eine Gruppe der Formel

$$CF_3\text{-}(CF_2)_a\text{-}O_b\text{-}(CF_2)_c\text{-}O_d\text{-}$$

ist,
wobei

a = 0, 1, 2 oder 3,
b = 0 oder 1,
c = 0, 1, 2 oder 3 und
d = 0 oder 1 ist.

4. Verfahren gemäß einem oder mehreren der Ansprüche 1 bis 3, **dadurch gekennzeichnet, dass** Rf‘ eine Gruppe der Formel

$$CF_3\text{-}(CF_2)_a\text{-}O_b\text{-}(CF_2)_c\text{-}O_d\text{-}(CR^1R^2)_e\text{-}(CR^3R^4)_f\text{-}O_g\text{-}$$

ist,
wobei

$R^1$, $R^2$, $R^3$ und $R^4$ unabhängig voneinander Wasserstoff oder eine Alkylgruppe sind,
a = 0, 1, 2 oder 3,
b = 0 oder 1,
c = 0, 1, 2 oder 3,
d = 0 oder 1,
e = 0, 1, 2, 3 oder 4,
f = 0, 1, 2, 3 oder 4 und
g = 0 oder 1 ist.

5. Verfahren gemäß einem oder mehreren der Ansprüche 1 bis 4, **dadurch gekennzeichnet, dass** Rf und Rf‘ gleich oder verschieden sind.

6. Verfahren gemäß einem oder mehreren der Ansprüche 1 bis 5, **dadurch gekennzeichnet, dass** Rf und/oder Rf‘ eine fluorierte C1-C6-Alkylgruppe ist, besonders bevorzugt eine perfluorierte C1-C4-Alkylgruppe, insbesondere eine perfluorierte C3-Alkylgruppe ist.

7. Verbindungen der Formel (I’)

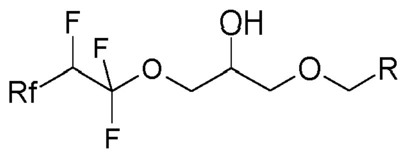

(I‘)

wobei

Rf eine fluorierte, lineare oder verzweigte, ggf. weitere Heteroatome enthaltende, Alkylgruppe ist,
R eine lineare oder verzweigte, ggf. Heteroatome enthaltende, Alkyl- oder Siloxangruppe oder eine Gruppe Rf‘ ist,
Rf‘ eine fluorierte, lineare oder verzweigte, ggf. weitere Heteroatome enthaltende Alkylgruppe ist,
und, wenn R eine Gruppe Rf‘ ist, Rf und Rf‘ verschieden sind.

8. Verbindungen gemäß Anspruch 7, **dadurch gekennzeichnet, dass** Rf eine Gruppe der Formel $CF_3\text{-}(CF_2)_{0\text{-}3}\text{-}$ oder $CF_3\text{-}(CF_2)_{0\text{-}3}\text{-}O\text{-}$ ist.

9. Verbindungen gemäß einem oder mehreren der Ansprüche 7 bis 8, **dadurch gekennzeichnet, dass** R eine C1-C20 Alkylgruppe ist.

10. Verbindungen gemäß einem oder mehreren der Ansprüche 7 bis 9, **dadurch gekennzeichnet, dass** Rf‘ eine Gruppe der Formel

$CF_3\text{-}(CF_2)_a\text{-}O_b\text{-}(CF_2)_c\text{-}O_d\text{-}(CR^1R^2)_e\text{-}(CR^3R^4)_f\text{-}O_g\text{-}$

ist,
wobei

$R^1$, $R^2$, $R^3$ und $R^4$ unabhängig voneinander Wasserstoff oder eine Alkylgruppe sind,
a = 0, 1, 2 oder 3,
b = 0 oder 1,
c = 0, 1, 2 oder 3,
d = 0 oder 1,
e = 0, 1, 2, 3 oder 4,
f = 0, 1, 2, 3 oder 4 und
g = 0 oder 1 ist.

**11.** Verwendung von Verbindungen der Formel (I) zur Herstellung von Verbindungen der Formel (VI)

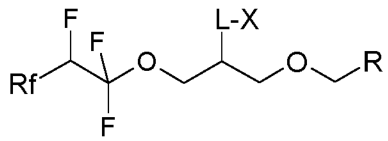

(VI)

wobei

Rf eine fluorierte, lineare oder verzweigte, ggf. weitere Heteroatome enthaltende, Alkylgruppe ist,
R eine lineare oder verzweigte, ggf. Heteroatome enthaltende, Alkylgruppe oder eine Gruppe Rf‘ ist,
Rf‘ eine fluorierte, lineare oder verzweigte, ggf. weitere Heteroatome enthaltende Alkylgruppe ist,
L eine Einfachbindung oder eine bivalente organische Gruppe ist und
X eine kationische, nichtionische, amphotere oder anionische Gruppe ist.

**12.** Verbindungen der Formel (VI‘)

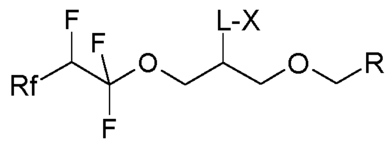

(VI‘)

wobei

Rf eine fluorierte, lineare oder verzweigte, ggf. weitere Heteroatome enthaltende, Alkylgruppe ist,
R eine C1-C20 Alkylgruppe ist,
L eine Einfachbindung oder eine bivalente organische Gruppe ist und
X eine kationische, nichtionische, amphotere oder anionische Gruppe ist.

**13.** Verbindungen gemäß Anspruch 12, **dadurch gekennzeichnet, dass** Rf eine Gruppe der Formel $CF_3\text{-}(CF_2)_{0\text{-}3}\text{-}$ oder $CF_3\text{-}(CF_2)_{0\text{-}3}\text{-}O\text{-}$ ist.

**14.** Verbindungen gemäß einem oder mehreren der Ansprüche 12 bis 13, **dadurch gekennzeichnet, dass** R eine C6-C14 Alkylgruppe ist.

**15.** Verwendung von Verbindungen gemäß einem oder mehreren der Ansprüche 12 bis 14 als Additive in Farben, Lacken, Druckfarben, Schutzanstrichen, Spezialcoatings in elektronischen oder optischen Anwendungen, Photolacken, Top Antireflective Coatings oder Bottom Antireflective Coatings, Entwicklerlösungen und Waschlösungen und Photoresists für photolithograqphische Verfahren, kosmetischen Produkten, Agrochemikalien, Bodenpolituren, photographischen Beschichtungen oder Beschichtungen optischer Elemente.

**16.** Mittel enthaltend eine oder mehrere Verbindungen gemäß einem oder mehreren der Ansprüche 12 bis 14 und einen für den jeweiligen Verwendungszweck geeigneten Träger sowie ggf. weitere spezifische Aktivstoffe.

**17.** Mittel nach Anspruch 16, **dadurch gekennzeichnet, dass** es sich bei dem Mittel um Farb- und Lackzubereitungen, Feuerlöschmittel, Schmierstoffe, Wasch- und Reinigungsmittel, Enteiser, Entwicklerlösungen und Waschlösungen und Photoresists für photolithograqphische Verfahren, kosmetische Produkte, Agrochemikalien, Bodenpolituren oder Hydrophobiermittel zur Textilausrüstung oder Glasbehandlung handelt.

## Claims

**1.** Process for the preparation of compounds of the formula (I)

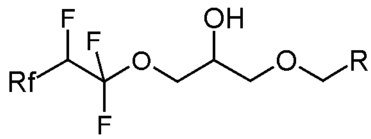

(I)

in which firstly compounds of the formula (II)

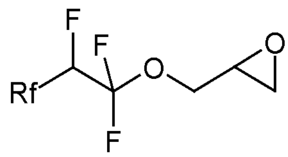

(II)

are prepared and these are then converted in a manner known per se into compounds of the formula (I), where

Rf is a fluorinated, linear or branched alkyl group, optionally containing further heteroatoms,

R is a linear or branched alkyl or siloxane group, optionally containing heteroatoms, or a group Rf', and

Rf' is a fluorinated, linear or branched alkyl group, optionally containing further heteroatoms.

**2.** Process according to Claim 1 comprising the steps:

a) reaction of compounds of the formula (III) with glycidol (IV) to give compounds of the formula (II)

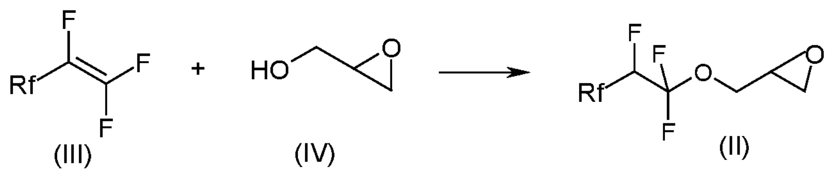

(III) (IV) (II)

and

b) reaction of compounds of the formula (II) with alcohols of the formula (V) to give compounds of the formula (I)

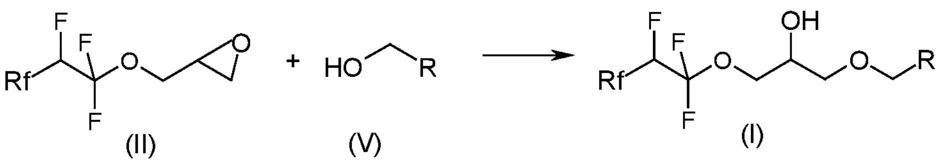

(II) (V) (I)

where

Rf is a fluorinated, linear or branched alkyl group, optionally containing further heteroatoms,

R is a linear or branched alkyl or siloxane group, optionally containing heteroatoms, or a group Rf', and Rf' is a fluorinated, linear or branched alkyl group, optionally containing further heteroatoms.

3. Process according to one or more of Claims 1 to 2, **characterised in that** Rf is a group of the formula

$$CF_3\text{-}(CF_2)_a\text{-}O_b\text{-}(CF_2)_c\text{-}O_d\text{-},$$

where

a = 0, 1, 2 or 3,
b = 0 or 1,
c = 0, 1, 2 or 3 and
d = 0 or 1 .

4. Process according to one or more of Claims 1 to 3, **characterised in that** Rf' is a group of the formula

$$CF_3\text{-}(CF_2)_a\text{-}O_b\text{-}(CF_2)_c\text{-}O_d\text{-}(CR^1R^2)_e\text{-}(CR^3R^4)_f\text{-}O_g\text{-},$$

where

$R^1$, $R^2$, $R^3$ and $R^4$ are, independently of one another, hydrogen or an alkyl group,
a = 0, 1, 2 or 3,
b = 0 or 1,
c = 0, 1, 2 or 3,
d = 0 or 1,
e = 0, 1, 2, 3 or 4,
f = 0, 1, 2, 3 or 4 and
g = 0 or 1.

5. Process according to one or more of Claims 1 to 4, **characterised in that** Rf and Rf' are identical or different.

6. Process according to one or more of Claims 1 to 5, **characterised in that** Rf and/or Rf' is a fluorinated C1-C6-alkyl group, particularly preferably a perfluorinated C1-C4-alkyl group, in particular a perfluorinated C3-alkyl group.

7. Compounds of the formula (I')

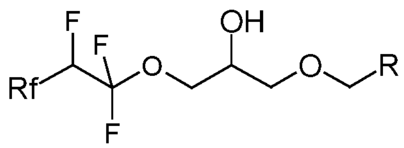

(I')

where

Rf is a fluorinated, linear or branched alkyl group, optionally containing further heteroatoms,
R is a linear or branched alkyl or siloxane group, optionally containing heteroatoms, or a group Rf',
Rf' is a fluorinated, linear or branched alkyl group, optionally containing further heteroatoms,
and, if R is a group Rf', Rf and Rf' are different.

8. Compounds according to Claim 7, **characterised in that** Rf is a group of the formula $CF_3\text{-}(CF_2)_{0\text{-}3}\text{-}$ or $CF_3\text{-}(CF_2)_{0\text{-}3}\text{-}O\text{-}$.

9. Compounds according to one or more of Claims 7 to 8, **characterised in that**
R is a C1-C20-alkyl group.

10. Compounds according to one or more of Claims 7 to 9, **characterised in that**
Rf' is a group of the formula

$$CF_3\text{-}(CF_2)_a\text{-}O_b\text{-}(CF_2)_c\text{-}O_d\text{-}(CR^1R^2)_e\text{-}(CR^3R^4)_f\text{-}O_g\text{-},$$

where

$R^1$, $R^2$, $R^3$ and $R^4$ are, independently of one another, hydrogen or an alkyl group,
a = 0, 1, 2 or 3,
b = 0 or 1,
c = 0, 1, 2 or 3,
d = 0 or 1,
e = 0, 1, 2, 3 or 4,
f = 0, 1, 2, 3 or 4 and
g = 0 or 1.

**11.** Use of compounds of the formula (I) for the preparation of compounds of the formula (VI)

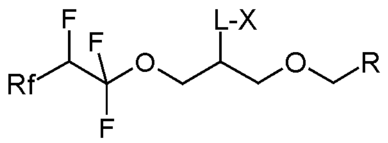

(VI)

where

Rf is a fluorinated, linear or branched alkyl group, optionally containing further heteroatoms,
R is a linear or branched alkyl group, optionally containing heteroatoms, or a group Rf',
Rf' is a fluorinated, linear or branched alkyl group, optionally containing further heteroatoms,
L is a single bond or a divalent organic group and
X is a cationic, nonionic, amphoteric or anionic group.

**12.** Compounds of the formula (VI')

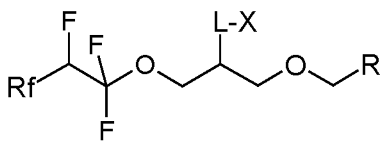

(VI')

where

Rf is a fluorinated, linear or branched alkyl group, optionally containing further heteroatoms,
R is a C1-C20-alkyl group,
L is a single bond or a divalent organic group and
X is a cationic, nonionic, amphoteric or anionic group.

**13.** Compounds according to Claim 12, **characterised in that** Rf is a group of the formula $CF_3\text{-}(CF_2)_{0\text{-}3}\text{-}$ or $CF_3\text{-}(CF_2)_{0\text{-}3}\text{-}O\text{-}$.

**14.** Compounds according to one or more of Claims 12 to 13, **characterised in that** R is a C6-C14-alkyl group.

**15.** Use of compounds according to one or more of Claims 12 to 14 as additives in paints, coatings, printing inks, protective coatings, specialty coatings in electronic or optical applications, photoresists, top antireflective coatings or bottom antireflective coatings, developer solutions and wash solutions and photoresists for photolithographic processes, cosmetic products, agricultural chemicals, floor polishes, photographic coatings or coatings of optical elements.

**16.** Compositions comprising one or more compounds according to one or more of Claims 12 to 14 and a vehicle which is suitable for the particular application, as well as optionally further specific active substances.

**17.** Compositions according to Claim 16, **characterised in that** the compositions are paint and coating preparations, fire extinguishing agents, lubricants, washing agents and detergents, deicers, developer solutions and wash solutions and photoresists for photolithographic processes, cosmetic products, agricultural chemicals, floor polishes or hydrophobicising agents for textile finishing or glass treatment.

## Revendications

**1.** Procédé de préparation de composés de formule (I)

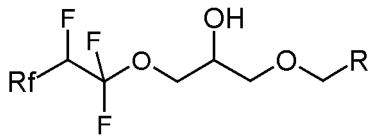

(I)

dans lequel, tout d'abord, des composés de formule (II)

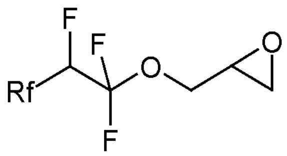

(II)

sont préparés et ceux-ci sont ensuite convertis, d'une manière connue en soi, en composés de formule (I), dans laquelle
Rf est un groupement alkyle linéaire ou ramifié, fluoré, contenant éventuellement d'autres hétéroatomes,
R est un groupement alkyle ou siloxane linéaire ou ramifié, contenant éventuellement des hétéroatomes, ou un groupement Rf', et Rf' est un groupement alkyle linéaire ou ramifié, fluoré, contenant éventuellement d'autres hétéroatomes.

**2.** Procédé selon la revendication 1, comprenant les étapes :

a) de réaction de composés de formule (III) avec du glycidol (IV) pour donner les composés de formule (II)

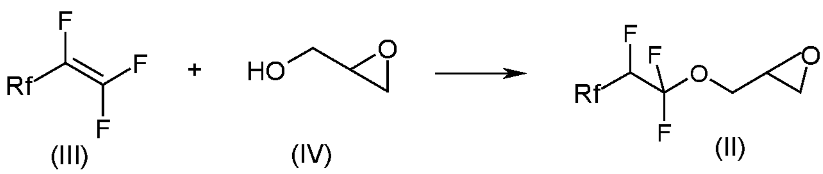

(III) (IV) (II)

et
b) de réaction de composés de formule (II) avec des alcools de formule (V) pour donner les composés de formule (I)

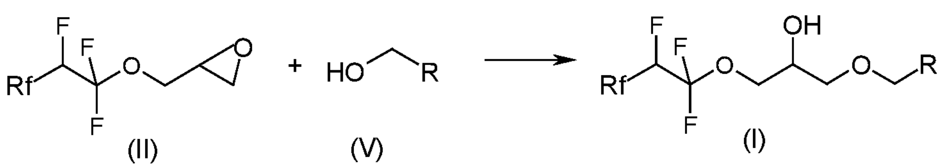

(II) (V) (I)

dans laquelle

Rf est un groupement alkyle linéaire ou ramifié, fluoré, contenant éventuellement d'autres hétéroatomes,
R est un groupement alkyle ou siloxane linéaire ou ramifié, contenant éventuellement des hétéroatomes, ou un groupement Rf', et
Rf' est un groupement alkyle linéaire ou ramifié, fluoré, contenant éventuellement d'autres hétéroatomes.

**3.** Procédé selon l'une ou plusieurs parmi les revendications 1 à 2, **caractérisé en ce que** Rf est un groupement de formule

$$CF_3\text{-}(CF_2)_a\text{-}O_b\text{-}(CF_2)_c\text{-}O_d\text{-},$$

dans laquelle

a = 0, 1, 2 ou 3,
b = 0 ou 1,
c = 0, 1, 2 ou 3 et
d = 0 ou 1.

**4.** Procédé selon l'une ou plusieurs parmi les revendications 1 à 3, **caractérisé en ce que** Rf' est un groupement de formule

$$CF_3\text{-}(CF_2)_a\text{-}O_b\text{-}(CF_2)_c\text{-}O_d\text{-}(CR^1R^2)_e\text{-}(CR^3R^4)_f\text{-}O_g\text{-},$$

dans laquelle

$R^1$, $R^2$, $R^3$ et $R^4$ sont, indépendamment les uns des autres, hydrogène ou un groupement alkyle,
a = 0, 1, 2 ou 3,
b = 0 ou 1,
c = 0, 1, 2 ou 3,
d = 0 ou 1,
e = 0, 1, 2, 3 ou 4,
f = 0, 1, 2, 3 ou 4 et
g = 0 ou 1.

**5.** Procédé selon l'une ou plusieurs parmi les revendications 1 à 4, **caractérisé en ce que** Rf et Rf' sont identiques ou différents.

**6.** Procédé selon l'une ou plusieurs parmi les revendications 1 à 5, **caractérisé en ce que** Rf et/ou Rf' est un groupement C1-C6-alkyle fluoré, particulièrement préférablement un groupement C1-C4-alkyle perfluoré, en particulier un groupement C3-alkyle perfluoré.

**7.** Composés de formule (I')

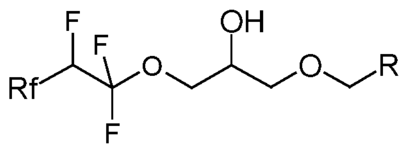

(I')

dans laquelle

Rf est un groupement alkyle linéaire ou ramifié, fluoré, contenant éventuellement d'autres hétéroatomes,
R est un groupement alkyle ou siloxane linéaire ou ramifié, contenant éventuellement des hétéroatomes, ou un groupement Rf',
Rf' est un groupement alkyle linéaire ou ramifié, fluoré, contenant éventuellement d'autres hétéroatomes,
et, si R est un groupement Rf', Rf et Rf' sont différents.

**8.** Composés selon la revendication 7, **caractérisés en ce que** Rf est un groupement de formule $CF_3\text{-}(CF_2)_{0\text{-}3}\text{-}$ ou

$CF_3-(CF_2)_{0-3}-O-$.

**9.** Composés selon l'une ou plusieurs parmi les revendications 7 à 8, **caractérisés en ce que** R est un groupement C1-C20-alkyle.

**10.** Composés selon l'une ou plusieurs parmi les revendications 7 à 9, **caractérisés en ce que** Rf' est un groupement de formule

$$CF_3-(CF_2)_a-O_b-(CF_2)_c-O_d-(CR^1R^2)_e-(CR^3R^4)_f-O_g-,$$

dans laquelle

$R^1$, $R^2$, $R^3$ et $R^4$ sont, indépendamment les uns des autres, hydrogène ou un groupement alkyle,
a = 0, 1, 2 ou 3,
b = 0 ou 1,
c = 0, 1, 2 ou 3,
d = 0 ou 1,
e = 0, 1, 2, 3 ou 4,
f = 0, 1, 2, 3 ou 4 et
g = 0 ou 1.

**11.** Utilisation de composés de formule (I) pour la préparation de composés de formule (VI)

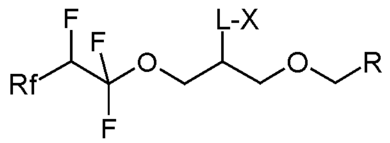

(VI)

dans laquelle

Rf est un groupement alkyle linéaire ou ramifié, fluoré, contenant éventuellement d'autres hétéroatomes,
R est un groupement alkyle linéaire ou ramifié, contenant éventuellement des hétéroatomes, ou un groupement Rf',
Rf' est un groupement alkyle linéaire ou ramifié, fluoré, contenant éventuellement d'autres hétéroatomes,
L est une liaison simple ou un groupement organique divalent et
X est un groupement cationique, non ionique, amphotère ou anionique.

**12.** Composés de formule (VI')

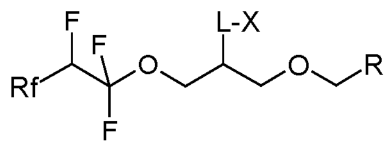

(VI')

dans laquelle

Rf est un groupement alkyle linéaire ou ramifié, fluoré, contenant éventuellement d'autres hétéroatomes,
R est un groupement C1-C20-alkyle,
L est une liaison simple ou un groupement organique divalent et
X est un groupement cationique, non ionique, amphotère ou anionique.

**13.** Composés selon la revendication 12, **caractérisés en ce que** Rf est un groupement de formule $CF_3-(CF_2)_{0-3}-$ ou $CF_3-(CF_2)_{0-3}-O-$.

14. Composés selon l'une ou plusieurs parmi les revendications 12 à 13, **caractérisés en ce que** R est un groupement C6-C14-alkyle.

15. Utilisation de composés selon l'une ou plusieurs parmi les revendications 12 à 14, comme additifs dans les peintures, les revêtements, les encres d'impression, les revêtements protecteurs, les revêtements spéciaux dans les applications électroniques ou optiques, les résines photosensibles, les revêtements antireflets supérieurs ou les revêtements antireflets inférieurs, les solutions de révélateur et les solutions de lavage ainsi que les photorésines pour les procédés photo-lithographiques, les produits cosmétiques, les substances agrochimiques, les encaustiques, les revêtements photographiques ou les revêtements d'éléments optiques.

16. Compositions comprenant un ou plusieurs composés selon l'une ou plusieurs parmi les revendications 12 à 14, et un véhicule qui est convenable pour l'application particulière, ainsi qu'éventuellement d'autres substances actives spécifiques.

17. Compositions selon la revendication 16, **caractérisées en ce que** les compositions sont des préparations de peinture et de revêtement, des agents d'extinction d'incendies, des lubrifiants, des agents de lavage et des détergents, des dégivreurs, des solutions de révélateur et des solutions de lavage ainsi que des photorésines pour les procédés photo-lithographiques, des produits cosmétiques, des substances agrochimiques, des encaustiques ou des agents d'hydrophobie pour la finition des textiles ou le traitement du verre.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2016069129 A1
- WO 2015124290 A1
- US 5608116 A


### In der Beschreibung aufgeführte Nicht-Patentliteratur


- **SOLOV'EV et al.** *The Journal of General Chemistry of the USSR,* 1991, vol. 61, 611-615